# EUROPEAN PATENT APPLICATION

(11) **EP 4 458 377 A1**
(43) Date of publication of application: **06.11.2024**
(21) Application number: 22916692.1
(22) Date of filing: 27.12.2022
(51) Int. Cl.: A61K 47/54, A61K 9/00, A61P 25/00

(54) **BLOOD-BRAIN BARRIER PENETRATING DRUG DELIVERY SYSTEM USING SIALYLLACTOSE**

(30) Priority: 27.12.2021 KR 20210188473
(71) Applicant: Genechem Inc., Daejeon 34025 (KR)
(72) Inventor: PARK, Eun Jung, Daejeon 34049 (KR); LEE, Sang Mi, Daejeon 34515 (KR); PARK, Dan Bi, Sejong 30064 (KR); KIM, Li La, Yeongdong-gun, Chungcheongbuk-do 29121 (KR); KIM, Dae Hee, Daejeon 35265 (KR)
(74) Representative: Mewburn Ellis LLP
(86) International application number: PCT/KR2022/021380
(87) International publication number: WO 2023/128551

(57) **Abstract**

Provided is a blood-brain barrier penetrating drug delivery system in which sialyllactose, which can penetrate the blood-brain barrier, is linked to a drug. The drug delivery system can deliver low-molecular weight small molecule drugs and high-molecular weight antibodies to the brain, and thus can be applied to various types of brain disease therapeutic agents or diagnostic substances.

## Description

### Technical Field

The present invention relates to a drug delivery system that penetrates the blood-brain barrier using sialyllactose.

### Background Art

The blood-brain barrier (BBB) is a cellular barrier composed of tight junctions having a high electrical resistance of 0.1 Ω·m or more between vascular endothelial cells interfaced with associated pericytes and astrocytes. The blood-brain barrier is a highly selective permeability barrier that separates circulating blood from brain extracellular fluid in the central nervous system (CNS), and serves as a gateway to regulate the entry and exit of substances.

The blood-brain barrier blocks bacteria, pathogens, and potentially dangerous substances in the blood, which can be transported through the blood, from being delivered to the brain, but it also blocks most central nervous system drugs from being delivered to the brain, and thus the drugs show low efficiency. To compensate for this, these drugs are administered at high doses, which may cause serious side effects in surrounding organs. Thus, to prevent ensure the therapeutic effect of drugs while preventing negative systemic effects, the discovery of an efficient drug delivery system that can penetrate the blood-brain barrier is required.

From this point of view, Roche, a Swiss pharmaceutical company, developed an antibody-based "brain shuttle". The brain shuttle binds to the transferrin receptor expressed in the epithelial cells of the brain blood vessels, is transported into the brain blood vessels, and then moves into the brain parenchyma. Roche is conducting a phase 3 clinical trial with gantenerumab, a monoclonal antibody targeting amyloid beta plaques which are a toxic protein in brain neurons that is suspected to be the cause of dementia. In this state, Roche is conducting a phase 1 clinical trial by redesigning a bispecific antibody obtained by attaching a brain shuttle to gantenerumab.

However, antibody-based drug delivery systems are not suitable for application to various types of drugs. Antibodies are high molecular substances that are much larger in size than small molecular substances such as small molecule compounds and peptides, and thus if one drug is attached to one antibody, the drug delivery efficiency decreases rather than increases. Therefore, as the drug that is attached to the antibody, a substance that has excellent efficacy but is difficult to use due to toxicity issues is used. To overcome these problems, studies have been conducted on various drug delivery systems using small molecule compounds, peptides, and aptamers, which have molecular weights smaller than antibodies.

Accordingly, the present inventors have found through experiments that a fluorescent substance attached to sialyllactose having a low molecular weight can penetrate the blood-brain barrier, and additionally have found that, when sialyllactose is connected to different substances, it can penetrate the blood-brain barrier, thereby completing the present invention.

### DISCLOSURE

### Technical Problem

An object to be achieved by the present invention is to provide a drug delivery system that can penetrate the blood-brain barrier using sialyllactose having a low molecular weight.

Objects to be achieved by the present invention are not limited to the object mentioned above, and other objects not mentioned above will be clearly understood by those skilled in the art from the following description.

### Technical Solution

In order to achieve the above object, in one embodiment of the present invention, one aspect of the present invention provides a blood-brain barrier-penetrating drug delivery system represented by Structural Formula 1 below:

[Structural Formula 1] SL-L-X

wherein
SL is sialyllactose or a salt thereof,
X is a biologically active substance to be delivered, and
L may be a bond or a chemical linker that connects SL to X.

In one embodiment, the sialyllactose may be 2,3-sialyllactose or 2,6-sialyllactose.

In one embodiment, the biologically active substance to be delivered is a biologically active substance for treating or diagnosing brain disease and may be selected from the group consisting of small molecule drugs, peptides, antibodies, proteins, natural or modified ssDNA, dsDNA, RNA, siRNA, and ASO.

In one embodiment, the chemical linker may be used without limitation as long as it has a structure that is structurally connected to both SL and X. For example, the chemical linker may be selected from the group consisting of -CONH-, -C(=O)-, -NH-, -O-, =N-, -SS-, and -N(CH₃)- and connected to SL, and may be selected from the group consisting of -CONH-, -C(=O)-, -NH-, -O-, =N-, -SS- and - N(CH₃)- and connected to X. A linkage between SL and X may have a structure selected from the group consisting of - (CH₂)a-(NHCO)b-(CH₂)c-, -(CH₂)a-(CONH)b-(CH₂)c-, -(CH₂)a-(CO)b-(CH₂)c-, -(CH₂)a-(NH)b-(CH₂)c-, and -(CH₂)a-(O)b-(CH₂)c-. Here, a may be an integer ranging from 0 to 10, b may be an integer of 0 or 1, and c may be an integer ranging from 0 to 10.

In one embodiment, the chemical linker may be a cleavable linker that promotes release of the biologically active substance to be delivered after the biologically active substance is delivered to the brain.

In one embodiment, the chemical linker may be bound to the hydroxyl group (-OH) of the lactose moiety of sialyllactose.

In one embodiment of the present invention, another aspect of the present invention provides a pharmaceutical composition for treating or diagnosing brain disease comprising the above-described blood-brain barrier-penetrating drug system.

### Advantageous Effects

The drug delivery system according to one embodiment of the present invention may be applied to drugs with low brain penetration efficiency to effectively deliver the drugs to the brain, and thus it may be used to treat and diagnose various brain diseases without toxicity problems caused by administration of high doses of drugs.

The effects of the present invention are not limited to the effects described above, and should be understood to include all effects that may be deduced from the configuration of the present invention described in the description of the invention or the appended claims.

### Brief Description of Drawings

FIG. 1 shows the results of an experiment conducted to comparing the brain penetration of lactose-bound Cy5.5 and sialyllactose-bound Cy5.5.
FIG. 2 shows the results of quantifying the fluorescence signal intensity of the brain portion shown in FIG. 1 and the results of measuring the fluorescence signal intensity in the brain extracted in the experiment shown in FIG. 1.
FIG. 3 shows the results of an experiment conducted to compare the brain penetration of sialic acid-bound Cy5.5 and sialyllactose-bound Cy5.5.
FIG. 4 shows the structural formula of sialyllactose-conjugated dopamine according to one embodiment of the present invention.
FIG. 5 shows the results of an experiment conducted to evaluate the brain penetration of 2,6-sialyllactose-conjugated dopamine in animals.
FIG. 6 shows the results of measuring the fluorescence signal of the brain portion in the experiment shown in FIG. 5 and the result of measuring the fluorescence signal in the extracted brain.
FIG. 7 shows the results of measuring the fluorescence signal in a cross section of the brain extracted from an animal administered 2,6-sialyllactose-conjugated dopamine.
FIG. 8 shows the results of an experiment conducted to evaluate the brain penetration of 2,3-sialyllactose-conjugated dopamine in animals.
FIG. 9 shows the results of measuring the fluorescence signal of the brain portion in the experiment shown in FIG. 8 and the result of measuring the fluorescence signal in the extracted brain.
FIG. 10 shows the structural formula of sialyllactose-conjugated methotrexate according to one embodiment of the present invention.
FIG. 11 shows the results of an experiment conducted to evaluate the brain penetration of sialyllactose-conjugated methotrexate in animals.
FIG. 12 shows the results of measuring the fluorescence signal of the brain portion in the experiment shown in FIG. 11 and the result of measuring the fluorescence signal in the extracted brain.
FIG. 13 shows the results of evaluating the anticancer effect of sialyllactose-conjugated methotrexate in a brain cancer animal model.
FIG. 14 shows the structural formula of sialyllactose-conjugated gemcitabine according to one embodiment of the present invention.
FIG. 15 shows the results of an experiment conducted to evaluate the brain penetration of sialyllactose-conjugated gemcitabine in animals.
FIG. 16 shows the results of an experiment conducted to evaluate the anticancer activity of sialyllactose-conjugated gemcitabine in a cell line.
FIG. 17 shows the results of an experiment conducted to evaluate the brain penetration of sialyllactose-conjugated antibody in animals.
FIG. 18 shows the results of measuring the fluorescence signal of the brain portion shown in FIG. 17.

### Best Mode

Hereinafter, the present invention will be described in detail.

The following description should be understood as an example to aid understanding of the present invention, and the spirit or scope of the present invention is not limited by the following description.

According to one aspect of the present invention, there may be provided a blood-brain barrier-penetrating drug delivery system represented by Structural Formula 1 below:

[Structural Formula 1] SL-L-X

wherein
SL is sialyllactose or a salt thereof, X is a drug to be delivered, and L may be a bond or a chemical linker that connects SL to X.

The sialyllactose is a type of human milk oligosaccharide (HMO) that is most abundant in colostrum of breast milk, and is a form in which sialic acid is attached to lactose. Depending on the position where sialic acid binds to lactose, there may be two types: 2,3-sialyllactose represented by Structural Formula 2 below, and 2,6-sialyllactose represented by Structural Formula 3 below.

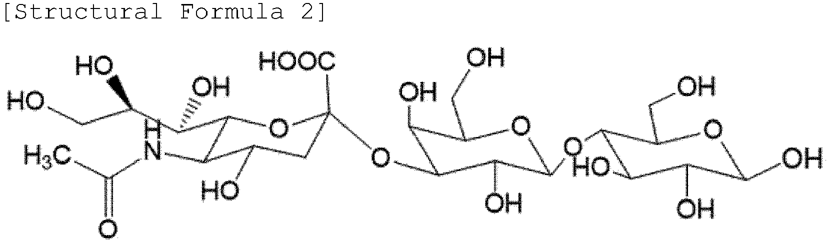

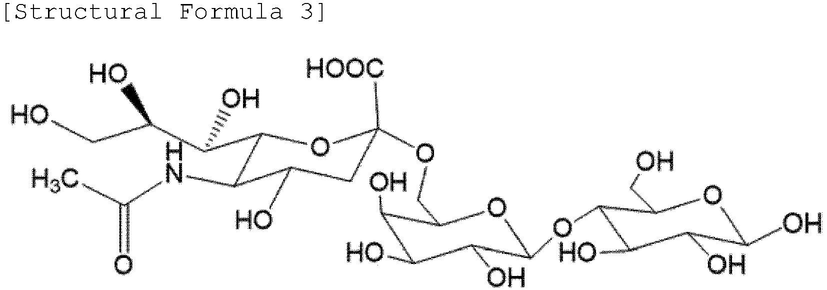

The sialyllactose may be extracted from breast milk, or synthesized chemically, or synthesized using an enzymatic reaction. Preferably, the sialyllactose may be synthesized using an enzymatic reaction which is capable of mass production and is environmentally friendly and safe because toxic catalysts or organic solvents are not used

The salt of sialyllactose may be Na, without being limited thereto.

The drug to be delivered may be a biologically active substance having a therapeutic effect against brain disease or a substance for diagnosis of brain disease, and may be selected from the group consisting of small molecule drugs, peptides, antibodies, proteins, natural or modified ssDNA, dsDNA, RNA, siRNA, and ASO.

Examples of the small molecule drugs include anticancer drugs such as cisplatin, carboplatin, melphalan, chlorambucil, dacarbazine, capecitabine, cytarabine, vinblastine, vincristine, paclitaxel, docetaxel, etoposide, topotecan, irinotecan, dactinomycin, doxorubicin, daunorubicin, mitomycin, bleomycin, temsirolimus, everolimus, lenalidomide, or pharmaceutically acceptable salts thereof; Alzheimer's disease therapeutic agents such as memantine, donepezil, galantamine, or pharmaceutically acceptable salts thereof; Parkinson's disease therapeutic agents such as pramipexole, ropinirole, amantadine, or pharmaceutically acceptable salts thereof; and neurotransmitters and brain function improving agents such as dopamine, acetylcholine, citicoline, choline alfoscerate (Alpha-GPC), or pharmaceutically acceptable salts thereof.

Examples of the antibodies include anticancer drugs such as trastuzumab, cetuximab, bevacizumab, pertuzumab, pembrolizumab, nivolumab, ipilimumab, atezolizumab, and durvalumab, and Alzheimer's disease therapeutic agents such as aducanumab.

Examples of the proteins or peptides include leuprolide or octreotide, nerve growth factor (NGF; brain-derived neurotrophic factor, BDNF; neurotrophin-3, NT-3), and transforming growth factor-β (TGF-β).

The brain disease may be a disorder or disease that occurs in or originates from the central nervous system. Examples of the brain disease include, but are limited to, brain tumor, cerebellar atrophy, Lou Gehrig's disease, stroke, Alzheimer's disease, dementia, and Parkinson's disease.

SL and X may be directly bound to each other. For example, the functional group of SL and the functional group may be directly bound to each other without a linker if the direct binding does not impair the blood-brain barrier penetration ability of SL and if the binding is degraded after penetration into the blood-brain barrier so that SL and X are separated from each other. Preferably, the hydroxyl group (-OH) of the lactose moiety of SL and a functional group that does not affect the physiological activity of X may be bound to each other.

In addition, SL and X may be connected to each other by a chemical linker. For example, the chemical linker may be bound to each of the functional group of SL and the functional group of X without impairing the blood-brain barrier penetration ability of SL and the physiological activity of X. Preferably, the chemical linker may be bound to each of the hydroxyl group (-OH) of the lactose moiety of SL and a functional group that does not affect the physiological activity of X.

The chemical linker that is used may have a structure that is structurally connected to both SL and X. The chemical linker may be selected from the group consisting of -CONH-, -C(=O)-, -NH-, -O-, =N-, -SS-, and -N(CH₃)- and connected to SL, and may be selected from the group consisting of -CONH-, -C(=O)-, -NH-, -O-, =N-, -SS- and - N(CH₃)- and connected to X. A linkage between SL and X may have a structure selected from the group consisting of - (CH₂)a-(NHCO)b-(CH₂)c-, -(CH₂)a-(CONH)b-(CH₂)c-, -(CH₂)a-(CO)b-(CH₂)c-, -(CH₂)a-(NH)b-(CH₂)c-, and -(CH₂)a-(O)b-(CH₂)c-. Here, a may be an integer ranging from 0 to 10, b may be an integer of 0 or 1, and c may be an integer ranging from 0 to 10.

The chemical linker may be a cleavable linker that promotes release of the biologically active substance to be delivered after the biologically active substance is delivered to the brain. Examples of the chemical linker include pH-sensitive linkers, peptidase-sensitive linkers, and photolabile linkers. Preferably, the chemical linker may be a peptidase-sensitive linker that is easily cleavable by peptidases present in cells. For example, the chemical linker may be selected from among those susceptible to cleavage by peptidases such as cathepsins B, C and D, which are peptidases preferentially expressed in tumor tissue.

The blood-brain barrier-penetrating drug system may be contained in a pharmaceutical composition for treating or diagnosing brain disease. The pharmaceutical composition may contain the blood-brain barrier-penetrating drug system alone, or may further contain at least one pharmaceutically acceptable carrier, excipient or diluent.

The "pharmaceutically acceptable" means that the composition is non-toxic to cells or humans exposed to the composition.

Furthermore, the pharmaceutical composition may be provided in combination with a conventionally known agent for treating or diagnosing brain disease. That is, the pharmaceutical composition may be administered in combination with a known compound that has a therapeutic or diagnostic effect on brain disease.

The term "administration" means introducing a predetermined substance into a subject by an appropriate method. The "subject" refers to any target animals such as rats, mice, and livestock, including humans, in which brain disease is to be treated or diagnosed. As a specific example, the subject may be a mammal, including a human.

The routes of administration of the pharmaceutical composition include, but are not limited to, oral, intravenous, intramuscular, intraarterial, intramedullary, intrathecal, intracardiac, transdermal, subcutaneous, intraperitoneal, intranasal, enteral, topical, sublingual or intrarectal routes.

The pharmaceutical composition may be administered orally or parenterally. For parenteral administration, external skin application, intraperitoneal injection, intrarectal injection, subcutaneous injection, intravenous injection, intramuscular injection, or intrathoracic injection is preferably selected, without being limited thereto. From the viewpoint of selecting a more effective absorption route, oral administration may be preferably selected.

For formulation, the pharmaceutical composition is formulated with commonly used diluents or excipients such as fillers, extenders, binders, wetting agents, disintegrants, and surfactants. Solid formulations for oral administration include tablets, pills, powders, granules, capsules, etc., and these solid formulations are prepared by mixing the extract with at least one excipient, such as starch, calcium carbonate, sucrose, lactose, or gelatin. In addition to simple excipients, lubricants such as magnesium stearate or talc may also be used. Liquid formulations for oral administration include suspensions, solutions, emulsions, and syrup, and may contain various excipients, for example, wetting agents, flavoring agents, aromatics and preservatives, in addition to water and liquid paraffin, which are frequently used simple diluents. Formulations for parenteral administration include sterilized aqueous solutions, non-aqueous solutions, suspensions, emulsions, freeze-dried preparations, and suppositories. As non-aqueous solvents or suspending agents, propylene glycol, polyethylene glycol, plant oils such as olive oil, injectable esters such as ethyl oleate, and the like may be used. As the base of the suppositories, witepsol, Macrogol, Tween 61, cacao butter, laurin fat, glycerogelatin and the like may be used.

### Mode for Invention

Hereinafter, the present invention will be described in more detail by way of examples and test examples. However, the following examples and test examples are for illustrating the present invention, and the scope of the present invention is not limited thereto.

### <Examples>

### 1. Experiment on Blood-Brain Barrier Penetration of Sialyllactose

In order to evaluate the possibility of applying sialyllactose to a blood-brain barrier-penetrating drug delivery system, an experiment on the brain penetration of sialic acid, lactose, and sialic lactose was conducted.

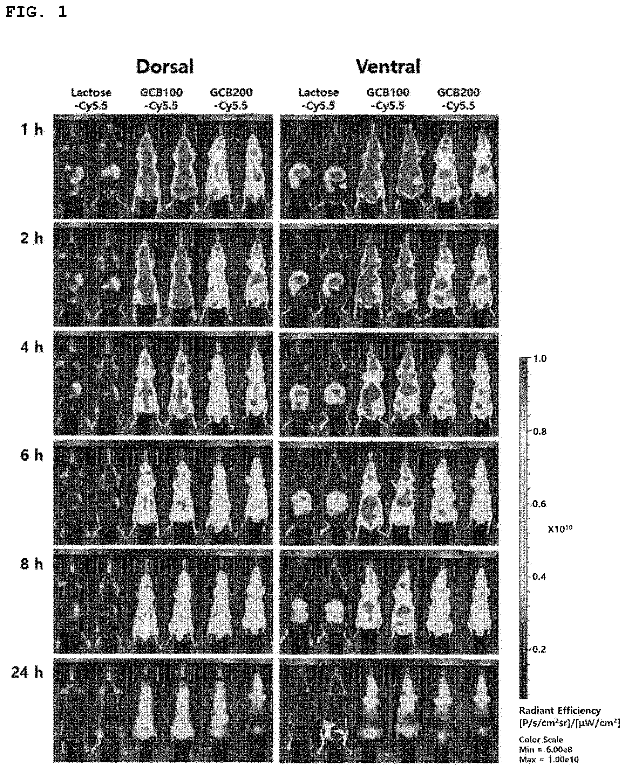

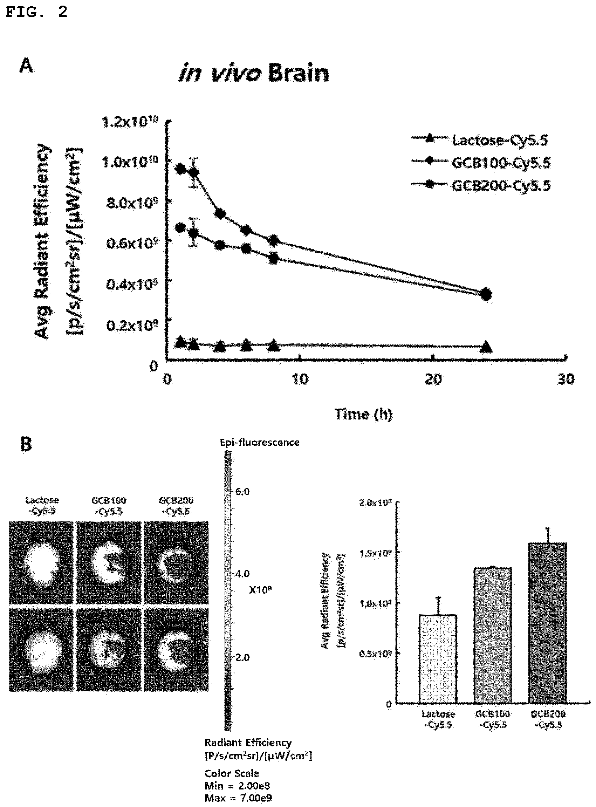

First, the fluorescent substance Cy5.5 (Genechem, Cat no. GCCD0064) was attached to each of lactose, 2,3-sialyllactose (GCB100) and 2,6-sialyllactose (GCB200), and each substance was dissolved and diluted in 10% DMSO, thus preparing samples (final DMSO concentration is approximately 2% of the total volume). After administering each sample at 1 µmol/kg into the veins of BALB/c nude mice, the fluorescence signals in the brains of the nude mice were measured at different time points (1, 2, 4, 6, 8, and 24 hours) using the IVIS spectrum CT (Perkin Elmer). 24 hours after administration, the nude mice were perfused with saline solution, the brains were extracted, and fluorescence present in the brains was measured (FIGS. 1 and 2). As a result, it was shown that Cy5.5 (control) barely penetrated the blood-brain barrier, and Cy5.5 attached to different sialyllactoses were all well delivered to the brain, compared to lactose-bound Cy5.5.

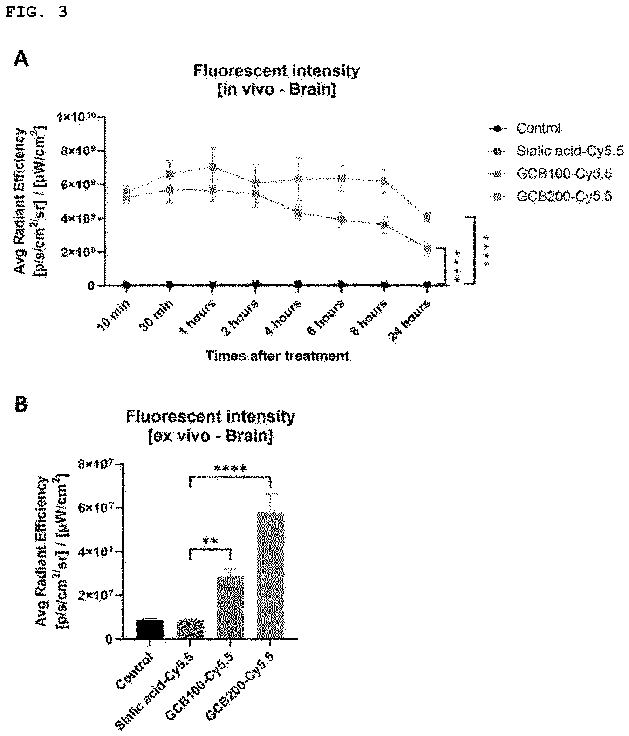

Next, the fluorescent substance Cy5.5 (Genechem, Cat no. GCCD0064) was attached to each of sialic acid, 2,3-sialyllactose (GCB100) and 2,6-sialyllactose (GCB200), and each substance was dissolved and diluted in 10% DMSO, thus preparing samples (final DMSO concentration is approximately 2% of the total volume). After administering each sample at 1 µmol/kg into the veins of BALB/c nude mice, the fluorescence signals in the brains of the nude mice were measured at different time points (10 min, 30 min, 1, 2, 4, 6, 8, and 24 hours) using the IVIS spectrum CT (Perkin Elmer). 24 hours after administration, the nude mice were perfused with saline solution, the brains were extracted, and fluorescence present in the brains was measured. As a result, as shown in FIG. 3, it was confirmed that Cy5.5 (control) barely penetrated the blood-brain barrier, and even when sialic acid was attached thereto, brain penetration remained at a similar level, whereas sialyllactose-attached Cy5.5 was well delivered to the brain, and in particular, the effect of 2,6-sialyllactose was higher than that of 2,3-sialyllactose.

Taken together, it could be confirmed that Cy5.5, which has a low brain penetration rate, penetrated the blood-brain barrier more easily when sialyllactose was attached thereto, and in particular, sialyllactose-attached Cy5.5 had a significantly higher brain penetration rate than sialic acid or lactose, which is a portion of sialyllactose.

### 2. In Vivo Experiment on Sialyllactose-Conjugated Dopamine

Parkinson's disease is a disease that occurs due to the loss of dopamine neurons. Currently, for the treatment of Parkinson's disease, L-dopa, a dopamine precursor that can penetrate the brain, is used as a therapeutic agent instead of dopamine, which has a low brain penetration rate, but it causes side effects such as levodopa-induced dyskinesia. Accordingly, an experiment was conducted to determine whether sialyllactose-conjugated dopamine could have increased brain penetration and replace L-dopa.

### 2-1) Production of Sialyllactose-Conjugated Dopamine

To produce GCB007 (2,3-sialyllactose-dopamine conjugate) and GCB008 (2,6-sialyllactose-dopamine conjugate), 5 g of dopamine was added to and dissolved in 50 ml of dimethylformamide and 11.6 ml of triethylamine, and then 8.95 g of MMTr-Cl was slowly added thereto while stirring at 0°C, followed by stirring for 2 hours. Then, the solution was concentrated and purified with silica gel to obtain 4.5 g of material. 1.3 g of the purified material was added to 0.06 g of 4-dimethylaminopyridine and dissolved in 100 ml of methylene chloride, and 0.6 g of 4-nitrophenyl chloroformate was added thereto, followed by stirring for 4 hours. After completion of the reaction, the methylene chloride layer was collected by fractionation with 100 ml of 0.5 M citric acid aqueous solution, treated with sodium sulfate, filtered through a glass filter, and then concentrated to obtain 2 g of material. 16 ml of dimethylformamide and 2 ml of triethylamine were added to 2 g of the material, followed by stirring. 0.54 g of each of aminohexyl-2,3-sialyllactose and 2,6-sialyllactose was added thereto, followed by stirring for 24 hours. The stirred product was concentrated and purified with silica gel to obtain 0.3 g of material. 3 ml of methanol was added to the obtained material and stirred, and then 6 ml of acetic acid was added thereto, followed by stirring for about 8 hours. After completion of the reaction, the reaction solution was concentrated and purified with silica gel to obtain GCB007 (2,3-sialyllactose-dopamine conjugate) and GCB008 (2,6-sialyllactose-dopamine conjugate).

To produce GCB009 (2,3-sialyllactose-dopamine conjugate) and GCB010 (2,6-sialyllactose-dopamine conjugate), 24 ml of dimethylformamide and 1 ml of pyridine were added to 1.2 g of each of carboxyl-2,3 sialyllactose and carboxyl-2,6 sialyllactose, followed by stirring. Then, 0.61 g of N-hydroxysuccinimide was added thereto, followed by stirring at 60°C for 90 minutes. The stirred solution was concentrated to obtain 1 g of material. Then, 0.67 g of the material obtained through the above process was added to 3.2 ml of dimethylformamide and 1.1 ml of triethylamine, followed by stirring. Then, 0.15 g of dopamine was added thereto, followed by stirring for 24 hours. Then, the stirred solution was concentrated and purified with silica gel to obtain 0.06 g of each of GCB009 (2,3-sialyllactose-dopamine conjugate) and GCB010 (2,6-sialyllactose-dopamine conjugate).

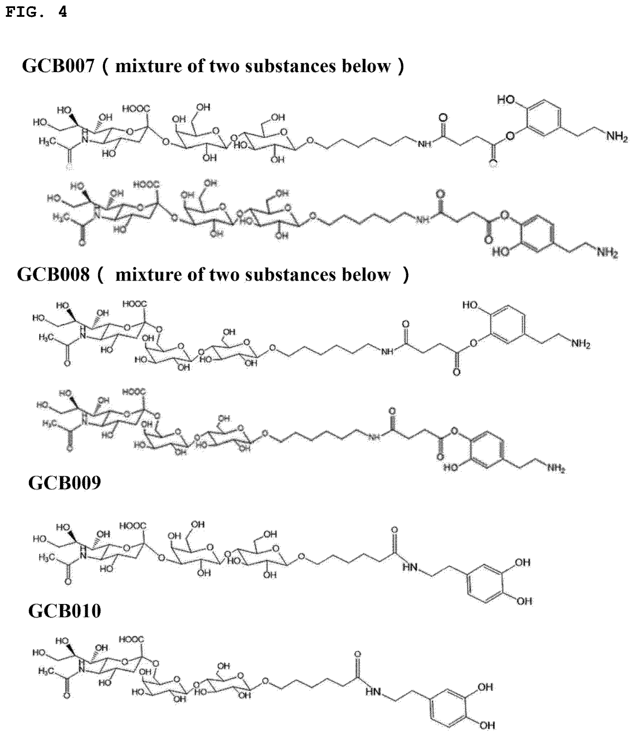

FIG. 4 shows the structures of GCB007 (2,3-sialyllactose-dopamine conjugate), GCB008 (2,6-sialyllactose-dopamine conjugate), GCB009 (2,3-sialyllactose-dopamine conjugate), and GCB010 (2,6-sialyllactose-dopamine conjugate).

### 2-2) Experiment on Blood-Brain Barrier Penetration of Sialyllactose-Conjugated Dopamine

Cy5.5, a fluorescent substance, was attached to each of dopamine, GCB007, and GCB008, and a blood-brain barrier penetration experiment was performed. The experiment was conducted in the same manner as the above-described experiment on blood-brain barrier penetration of sialyllactose.

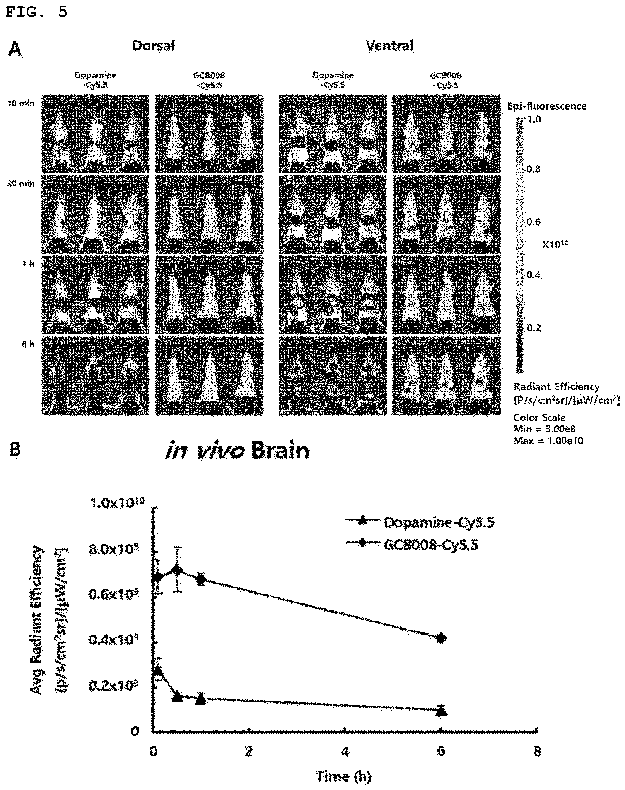

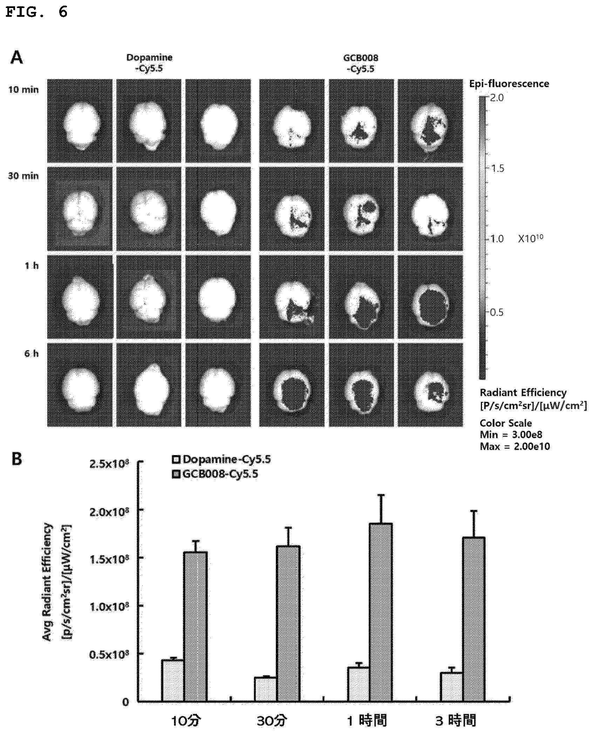

First, the time-dependent *in vivo* fluorescence distribution of Cy5.5-attached dopamine and Cy5.5-attached GCB008 and the fluorescence signal thereof in the brain were measured (FIG. 5). In addition, the brains were extracted at different time points and the fluorescence distribution and fluorescence signals in the brains were measured (FIG. 6). As a result, it was confirmed that sialyllactose-attached dopamine had a higher brain penetration rate than dopamine to which sialyllactose was not attached.

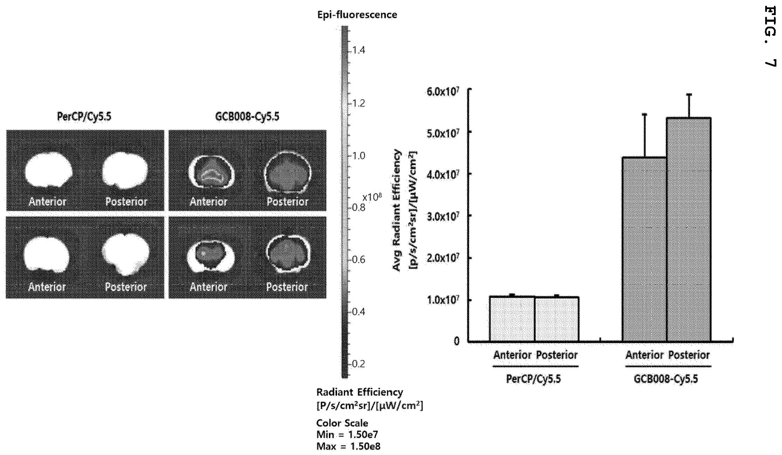

Additionally, to determine whether the fluorescent material is distributed not only throughout the entire region but also in the brain cross-section, PerCP/Cy5.5 (Abcam, Cat no. ab102911) was used as a control material, and Cy5.5-attached GCB008 was administered, and after 24 hours, the brain was extracted and sectioned, and the fluorescence distribution in the brain cross-section was measured. As a result, as shown in FIG. 7, it could be confirmed that sialyllactose-conjugated dopamine was detectable even in the brain cross section.

Next, in order to confirm that the brain penetration phenomenon was not a delivery effect caused by the fluorescent material Cy5.5, an experiment on the blood-brain barrier penetration of Cy5.5-attached GCB007 was conducted using Cy5.5 as a control.

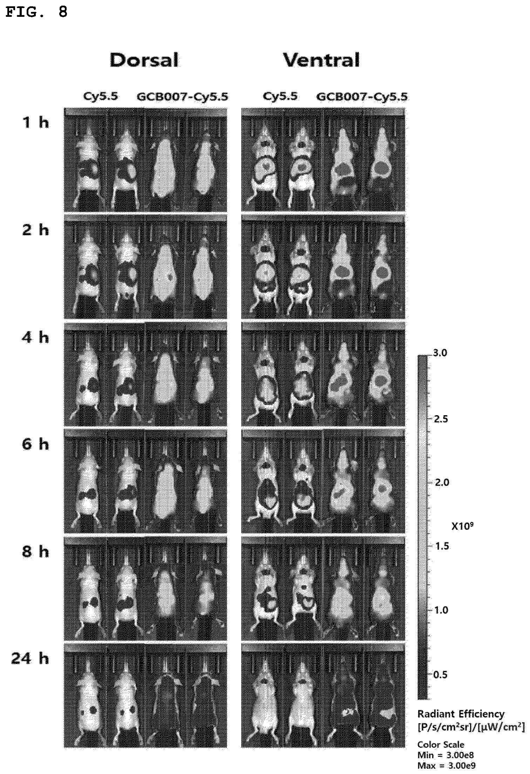

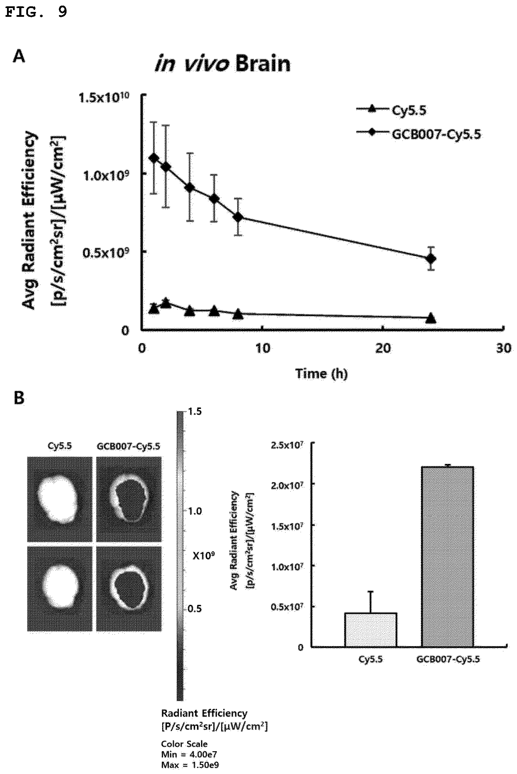

The time-dependent *in vivo* fluorescence distribution and fluorescence signal of each of Cy5.5 and Cy5.5-attached GCB007 were measured (FIG. 8), and after 24 hours, the brain was extracted, the fluorescence distribution and fluorescence signal in the brain were measured (FIG. 9). As a result, it could be confirmed that Cy5.5 was barely detected in the brain, whereas GCB007-Cy5.5 was clearly detected, indicating that brain penetration of sialyllactose-conjugated dopamine was induced by sialyllactose, not by Cy5.5.

### 3. In Vivo Experiment on Sialyllactose-Conjugated Methotrexate

### 3-1) Production of Sialyllactose-Conjugated Methotrexate

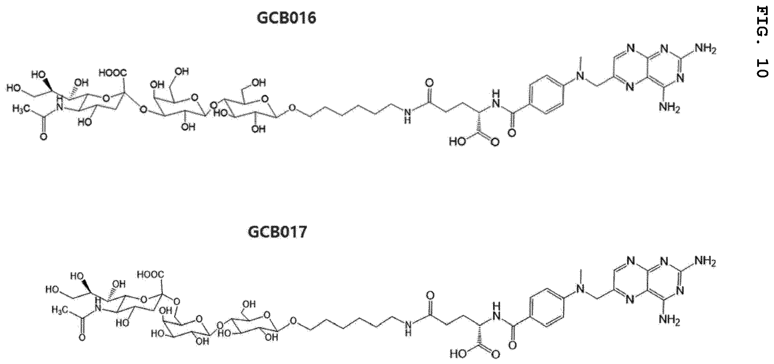

10 ml of pyridine and 0.337 ml of triethylamine were added to 1 g of methotrexate, followed by stirring. Then, 0.027 g of 4-dimethylaminopyridine and 0.887 g of 4-nitrophenyl chloroformate were slowly added thereto, followed by stirring for 4 hours. After completion of the reaction, the reaction solution was concentrated to obtain 1 g of material. 15 ml of dimethylformamide and 2.42 ml of triethylamine were added to 1 g of the obtained material, followed by stirring. Then, 0.63 g of each of aminohexyl-2,3-sialyllactose and aminohexyl-2,6-sialyllactose was added thereto, followed by stirring for 12 hours. After completion of the reaction, the reaction solution was concentrated and purified with silica to obtain GCB016 (2,3-sialyllactose-methotrexate conjugate) and GCB017 (2,6-sialyllactose-methotrexate conjugate). FIG. 10 shows the structures of GCB016 (2,3-sialyllactose-methotrexate conjugate) and GCB017 (2,6-sialyllactose-methotrexate conjugate).

### 3-2) Experiment on Blood-Brain Barrier Penetration of Sialyllactose-Conjugated Methotrexate

Cy5.5 was attached to methotrexate and used as a control material, and an experiment on the blood-brain barrier penetration each of the control material and Cy5.5-attached GCB017 was conducted.

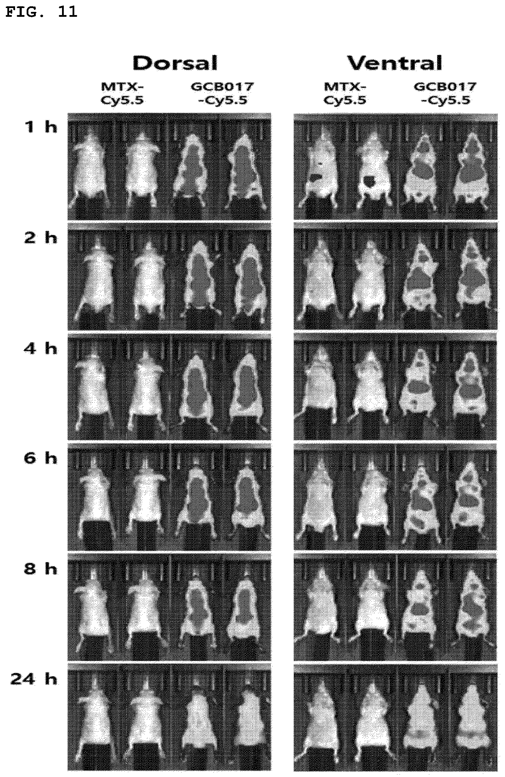

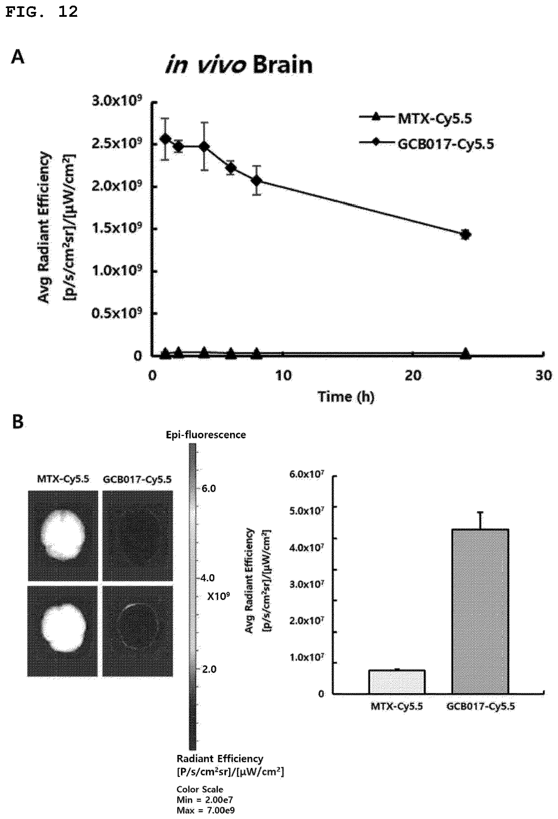

The time-dependent fluorescence distribution of each of Cy5.5-attached methotrexate and Cy5.5-GCB017 and the fluorescence signal thereof in the brain were measured (FIGS. 11 and 12A, and the fluorescence distribution image and fluorescence signal in the brain extracted 24 hours after administration were measured (FIG. 12B). As a result, it could be confirmed that, when methotrexate was administered, almost no fluorescence signal appeared in the brain, whereas when sialyllactose-conjugated methotrexate was administered, a strong fluorescence signal appeared, indicating that sialyllactose could induce the delivery of methotrexate to the brain when bound to methotrexate.

### 3-3) Experiment on Brain Tissue Penetration and Substance Dissociation of Sialyllactose-Conjugated Methotrexate

To examine the distribution and dissociation of sialyllactose-conjugated methotrexate in tissues, GCB017 was administered and the presence of GCB017 and its dissociates (linker-6'SL (L-6'SL) and linker) in the brain and pancreas was checked. Specifically, GCB017 was dissolved and diluted in 10% DMSO, thus preparing a sample (final DMSO concentration was approximately 2% of the total volume). The sample was administered intravenously (IV) at a dose of 80 mg/kg or administered orally (PO) at a dose of 80 mg/kg. Then, at different time points (0, 0.5, 2, and 4 hours), the animals were anesthetized, heart blood was collected, saline solution was perfused, and the brain and pancreas were extracted. Each of the extracted tissues was homogenized in 50 mM Tris-HCl buffer (pH 7.5) at a ratio of 1:5 and centrifuged at 3,000 rpm and 4°C for 20 minutes, and the supernatant was collected and subjected to mass spectrometry (SCIEX Triple Quad 3500 LC-MS/MS system).

**[Table 1]**

| (ng/ml) | | | | Brain | | |
|---|---|---|---|---|---|---|
| | | | | GCB017 | L-6'SL | Linker |
| Vehicle | | G1 | 101 | N/D | N/D | N/D |
| | | | 102 | N/D | N/D | N/D |
| GCB017 (80 mg/kg, IV) | 0.5h | C2 | 201 | 557.75 | N/D | N/D |
| | | | 202 | 246.04 | N/D | N/D |
| | | | 203 | 82.46 | N/D | N/D |
| | 2h | C3 | 301 | 52.73 | N/D | N/D |
| | | | 302 | 66.80 | N/D | N/D |
| | | | 303 | 41.10 | N/D | N/D |
| | 4h | C4 | 401 | 39.94 | N/D | N/D |
| | | | 402 | 57.44 | N/D | N/D |
| | | | 403 | 161.80 | N/D | N/D |
| GCB017 (80 mg/kg, PO) | 0.5h | G5 | 501 | 34.78 | N/D | N/D |
| | | | 502 | 169.99 | N/D | N/D |
| | 2h | C6 | 601 | N/D | N/D | N/D |
| | | | 602 | N/D | N/D | N/D |
| | 4h | C7 | 701 | 30.34 | N/D | N/D |
| | | | 702 | N/D | N/D | N/D |

As a result, as shown in Table 1 above, it was confirmed that GCB017 and its dissociates were detected in the brain tissue not only when GCB017 was administered intravenously but also when administered orally. This means that methotrexate, which is rarely delivered to the brain, can be delivered across the blood-brain barrier when conjugated with sialyllactose, and is then dissociated in the brain.

### 3-4) Evaluation of Efficacy of Sialyllactose-Conjugated Methotrexate in Brain Cancer Animal Model

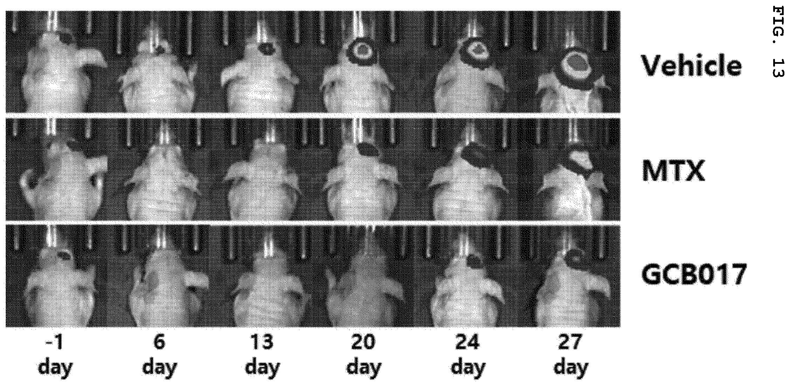

In order to examine whether GCB017 is delivered to the brain and exhibits the anticancer effect of methotrexate, a mouse brain cancer model was produced and the evaluation of anticancer efficacy was performed. The brain cancer model was produced by transplanting U-87MG, a brain cancer cell line that expresses luciferase. For transplantation into the brain, each animal was anesthetized by isoflurane injection and fixed on a stereotaxic arm, and the head skin was incised vertically by about 1 cm and the injection site was exposed using a micro drill. The cell line was transferred into a 25-µl Hamilton syringe equipped with a 31-gauge needle, and then the cell line was transplanted at a rate of 1 µl/min to an area 1 mm anterior, 2 mm lateral, and 3 mm deep based on bregma. After cell line transplantation, in order to prevent reflux and ensure good absorption, the syringe was maintained for about 10 minutes and separated, and the incision was sutured and disinfected. After confirming that the anesthesia had worn off, the animals were reared normally. Starting 1 week after model production, each of methotrexate and GCB017 was administered intravenously twice a week for a total of 8 times for 4 weeks, and the size of the brain tumor was measured by optical imaging (FIG. 13). It was confirmed that, in the case of the negative control group (vehicle), the tumor was noticeably large on day 20 and tended to increase as time passed to days 24 and 27, and in the case of the positive control group administered methotrexate, the tumor size also tended to increase gradually starting from day 20. On the other hand, it could be confirmed that, when compared to the negative control group and positive control group, in the case of the experimental group administered GCB017 at a dose of 22 µmol/kg, tumor formation was delayed and the tumor size was also controlled. In combination with the results of Examples 3-2 and 3-3 above, it is believed that the sialyllactose-methotrexate conjugate according to the present invention has an improved BBB penetration ability compared to methotrexate and inhibits tumor formation and growth.

### 4. Experiment on Blood-Brain Barrier Penetration of Sialyllactose-Conjugated Gemcitabine

### 4-1) Production of Sialyllactose-Conjugated Gemcitabine

**Production of 2,3-sialyllactose-gemcitabine conjugate:** 25 ml of dimethylformamide and 0.89 ml of triethylamine were added to 1.4 g of gemcitabine, followed by stirring. Then, 0.94 g of imidazole and 1.44 g of TBDMS-Cl were added thereto, followed by stirring for 4 hours. The stirred solution was concentrated and purified with silica gel to obtain 1.9 g of material. 12.5 ml of pyridine and 0.6 g of 4-dimethylaminopyridine were added to the obtained material, followed by stirring. Then, 1.39 ml of ditertiary butyl dicarbonate was slowly added thereto. The resulting solution was stirred, concentrated, fractionated with 0.5 M citric acid aqueous solution and methylene chloride, treated with sodium sulfate, filtered through a glass filter, concentrated, and then purified to obtain 1.2 g of material which was then dissolved in 8 ml of pyridine. 0.03 g of 4-dimethylaminopyridine and 0.327 g of succinic anhydride were added thereto, followed by stirring. The stirred solution was concentrated and fractionated with 0.5 M aqueous citric acid solution and methylene chloride. The resulting fraction was treated with sodium sulfate, filtered through a glass filter, concentrated, and then purified to obtain 0.6 g of material. The obtained material and 0.013 g of 4-dimethylaminopyridine were dissolved in 6 ml of pyridine and 0.42 ml of triethylamine. 0.248 g of 4-nitrophenyl chloroformate was added to the solution, followed by stirring for 4 hours. After completion of the reaction, the reaction solution was fractionated with 0.5 M citric acid aqueous solution and 100 ml of methylene chloride. The methylene chloride layer was collected, treated with sodium sulfate, filtered through a glass filter, and then concentrated to obtain 0.6 g of material. Then, 6 ml of dimethylformamide and 0.62 ml of triethylamine were added to 0.6 g of the obtained material, followed by stirring. Then, 0.5 g of aminohexyl-3'SL was added thereto, followed by stirring. The stirred solution was concentrated and purified with silica gel to obtain 0.25 g of material. Then, 5 ml of distilled water and 0.025 ml of acetic acid were added to the obtained material, followed by stirring at 60°C for 24 hours. Then, the stirred solution was concentrated and purified with silica gel to obtain gemcitabine-succinate-aminohexyl-3'SL (GCB033).

**Production of 2,6-Sialyllactose-Gemcitabine Conjugate:** 41 ml of dimethylformamide and 1.45 ml of triethylamine were added to 2.28 g of gemcitabine, followed by stirring, and then 1.53 g of imidazole and 2.35 g of TBDMS-Cl were added thereto, followed by stirring for 4 hours. The stirred solution was concentrated and purified with silica gel to obtain 1.9 g of material. 3.75 g of the material obtained in this way was added to 50 ml of pyridine and 1.2 g of 4-dimethylaminopyridine, followed by stirring. Then, 2.7 ml of di-tert-butyl dicarbonate was slowly added thereto, followed by stirring. The stirred solution was concentrated and fractionated with 0.5 M citric acid aqueous solution and methylene chloride. The resulting material was treated with sodium sulfate, filtered through a glass filter, concentrated, and then purified to obtain 3.2 g of material which was then dissolved in 21 ml of pyridine. 0.082 g of 4-dimethylaminopyridine and 0.87 g of succinic anhydride were added thereto, followed by stirring. The stirred solution was concentrated and fractionated with 0.5 M citric acid aqueous solution and methylene chloride. The resulting material was treated with sodium sulfate, filtered through a glass filter, concentrated, and then purified to obtain 2.9 of material. 2.9 of the obtained material and 0.061 g of 4-dimethylaminopyridine were dissolved in 29 ml of pyridine and 2.5 ml of triethylamine, and then 1.21 g of 4-nitrophenyl chloroformate was added thereto, followed by stirring for 4 hours. After completion of the reaction, the reaction solution was fractionated with 0.5 M citric acid aqueous solution and 100 ml of methylene chloride. The methylene chloride layer was collected, treated with sodium sulfate, filtered through a glass filter, and then concentrated to obtain 2.9 g of material to which 29 ml of dimethylformamide and 3 ml of triethylamine were added, followed by stirring. Then, 2.4 g of aminohexyl-6'SL was added to the stirred solution, followed by stirring. Then, the stirred solution was concentrated and purified with silica gel to obtain 1.2 g of material to which 24 ml of distilled water and 0.12 ml of acetic acid were added, followed by stirring at 60°C for 24 hours. Then, the stirred solution was concentrated and purified with silica gel to obtain to gemcitabine-succinate-aminohexyl-6'SL (GCB034).

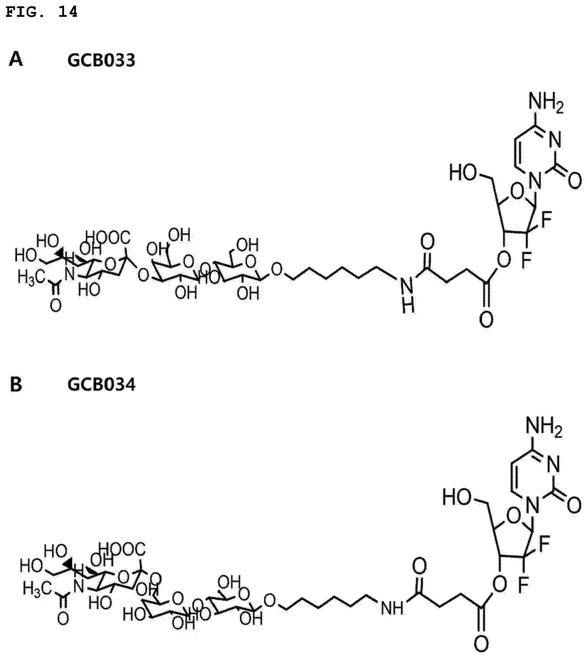

FIG. 14 shows the structures of GCB033 (2,3-sialyllactose-gemcitabine conjugate) and GCB034 (2,6-sialyllactose-gemcitabine conjugate) produced as described above.

### 4-2) Experiment on Blood-Brain Barrier Penetration of Sialyllactose-Conjugated Gemcitabine

To evaluate the blood-brain barrier penetration of the gemcitabine conjugates, Cy5.5 was attached to each of GCB033 and GCB034, and an experiment on blood-brain barrier penetration was performed. Here, Cy5.5 alone and gemcitabine attached to only Cy5.5 were used as controls. First, each material was dissolved and diluted in 10% DMSO, thus preparing samples (final DMSO concentration is approximately 2% of the total volume). After administering each sample at 1 µmol/kg into the veins of BALB/c nude mice, the fluorescence signals in the brains of the nude mice were measured at different time points (10 min, 30 min, 1, 2, 4, 6, 8, and 24 hours) using the IVIS spectrum CT (Perkin Elmer). 24 hours after administration, the nude mice were perfused with saline solution, the brains were extracted, and fluorescence present in the brains was measured.

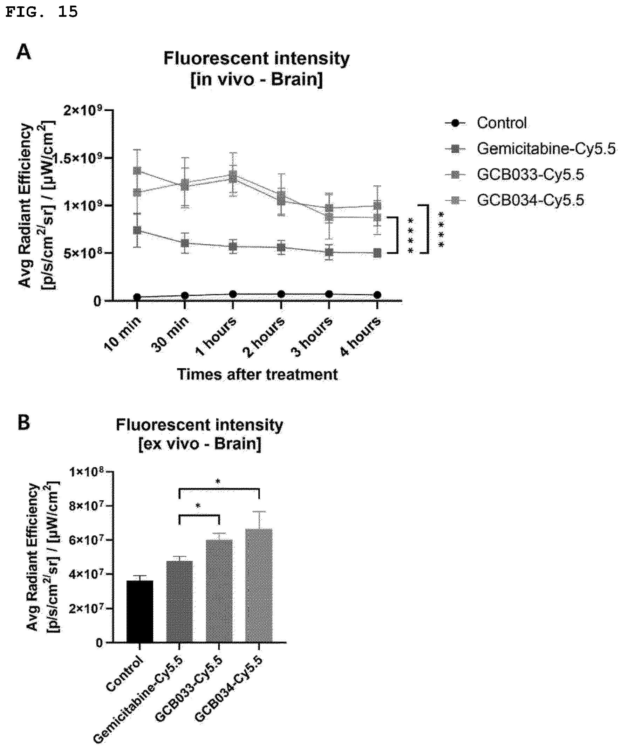

As a result, as shown in FIG. 15, it could be confirmed that when Cy5.5 attached to sialyllactose-conjugated gemcitabine was administered, a stronger fluorescence signal compared to when Cy5.5-gemcitabine was administered appeared, indicating that the brain penetration rate of gemcitabine was improved by sialylactose.

### 4-3) Experiment on Brain Tissue Penetration and Substance Dissociation of Sialyllactose-Conjugated Gemcitabine

To examine the distribution and dissociation of sialyllactose-conjugated gemcitabine in tissues, GCB034 was administered and the presence of GCB034 and its dissociates (linker-6'SL (L-6'SL) and linker) in the brain and pancreas was checked. Specifically, GCB034 was dissolved and diluted in 10% DMSO, thus preparing a sample (final DMSO concentration was approximately 2% of the total volume). The sample was administered intravenously (IV) at a dose of 80 mg/kg or administered orally (PO) at a dose of 246 mg/kg. Then, at different time points (0, 0.5, 2, and 4 hours), the animals were anesthetized, heart blood was collected, saline solution was perfused, and the brain and pancreas were extracted. Each of the extracted tissues was homogenized in 50 mM Tris-HCl buffer (pH 7.5) at a ratio of 1:5 and centrifuged at 3,000 rpm and 4°C for 20 minutes, and the supernatant was collected and subjected to mass spectrometry (SCIEX Triple Quad 3500 LC-MS/MS system).

**[Table 2]**

| (ng/ml) | | | Brain | | | |
|---|---|---|---|---|---|---|
| | | | GCB034 | L-6'SL | Linker | |
| Vehicle | G1 | 101 | N/D | N/D | N/D | |
| | | 102 | N/D | N/D | N/D | |
| | | 103 | N/D | N/D | N/D | |
| GCB034 (82 mg/kg, IV) | 0.5h | C2 | 201 | N/D | N/D | N/D |
| | | | 202 | 137.90 | 5.42 | N/D |
| | | | 203 | N/D | N/D | N/D |
| | 2h | C3 | 301 | N/D | N/D | N/D |
| | | | 302 | 62.21 | N/D | N/D |
| | | | 303 | 66.96 | 5.49 | N/D |
| | 4h | C4 | 401 | N/D | N/D | N/D |
| | | | 402 | N/D | N/D | N/D |
| | | | 403 | N/D | N/D | N/D |

As a result, as shown in Table 2 above, it was confirmed that, when GCB034 was administered intravenously, GCB017 penetrated the BBB and the presence of GCB034 and its dissociates were detected in the brain and pancreas, suggesting that sialyllactose-conjugated gemcitabine was delivered to the brain and then could be dissociated in the brain.

### 4-4) Experiment for Evaluating Anticancer Activity of Sialyllactose-Conjugated Gemcitabine

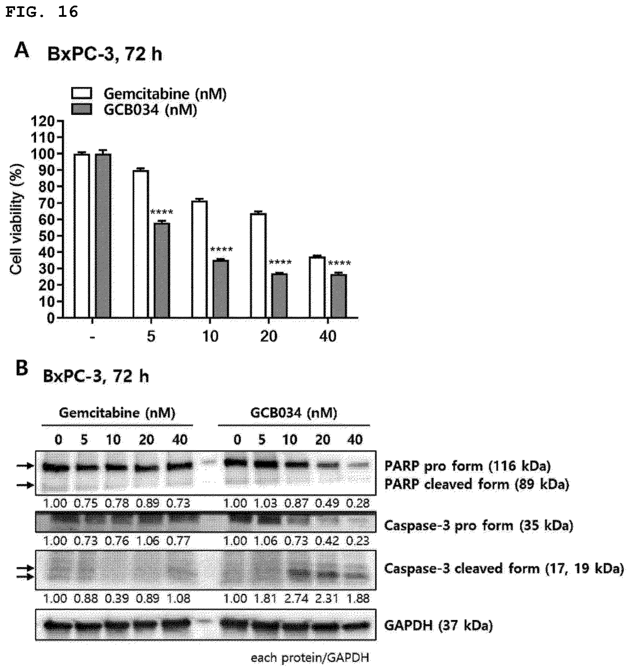

To evaluate the effect of conjugation with sialyllactose on the anticancer activity of gemcitabine, which is used as an anticancer drug, a cancer cell line (BxPC-3) was treated with each of GCB034 and gemcitabine at different concentrations (5, 10, 20, and 40 nM), and after 72 hours, the cell viability was analyzed, and apoptosis markers PARP and Caspase-3 were checked. As a result, it could be confirmed that GCB034 could not only induce cancer cell death at all treatment concentrations, but also exhibited a higher anticancer effect than gemcitabine to which sialyllactose was not conjugated (FIG. 16A). Corresponding to these results, it could be confirmed that the decrease in the pro-form of PARP and the increase in Caspase-3 cleavage that appear as apoptosis progresses were significant in a concentration-dependent manner for the gemcitabine conjugate (FIG. 16B).

### 5. Experiment on Blood-Brain Barrier Penetration of Sialyllactose-Conjugated Antibody

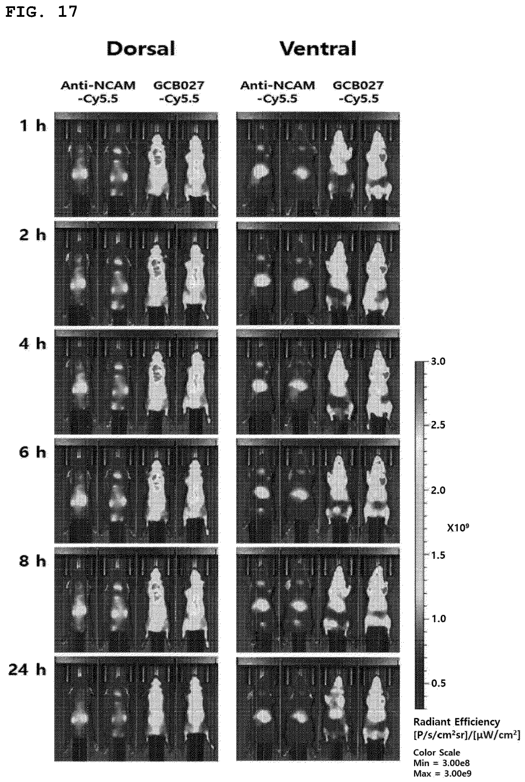

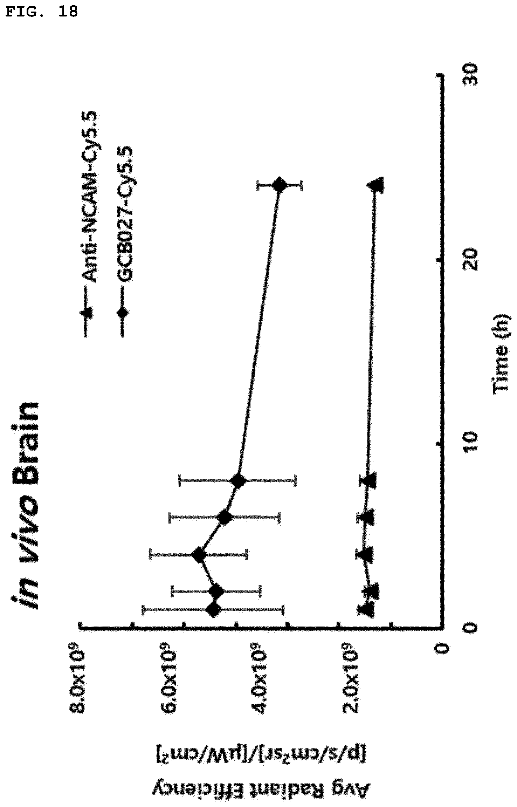

GCB027 (sialyllactose + anti-NCAM) was produced by connecting anti-NCAM antibody (14-0567-82, eBioscience) to sialyllactose using SH-linker (3-(2-pyridyldithio)propionic acid N-hydroxysuccinimide ester, Sigma-Aldrich, P3415). Cy5.5 was attached to the anti-NCAM antibody and used as a control material, and an experiment of blood-brain barrier penetration of each of the control material and Cy5.5-attached GCB027 was performed. As a result of measuring the time-dependent *in vivo* fluorescence distribution of each of Cy5.5-attached anti-NCAM and Cy5.5-attached GCB027 and the fluorescence signal thereof in the brain (FIGS. 17 and 18), it could be confirmed that even the antibody with a large molecular weight could be easily delivered to the brain when sialyllactose was attached thereto.

The above description of the present invention is exemplary, and those of ordinary skill in the art will appreciate that the present invention can be easily modified into other specific forms without departing from the technical spirit or essential characteristics of the present invention. Therefore, it should be understood that the exemplary embodiments described above are exemplary in all aspects and are not restrictive. For example, each component described to be of a single type can be implemented in a distributed manner. Likewise, components described to be distributed can be implemented in a combined manner.

The scope of the present invention is defined by the following claims, and it shall be understood that all modifications and embodiments conceived from the meaning and scope of the claims and their equivalents are included in the scope of the present invention.

## Claims

1. A blood-brain barrier-penetrating drug delivery system represented by Structural Formula 1 below:
[Structural Formula 1] SL-L-X
wherein
SL is sialyllactose or a salt thereof,
X is a drug to be delivered, and
L is a bond or a chemical linker that connects SL to X.

2. The blood-brain barrier-penetrating drug delivery system of claim 1, wherein the sialyllactose is 2,3-sialyllactose or 2,6-sialyllactose.

3. The blood-brain barrier-penetrating drug delivery system of claim 1, wherein the drug to be delivered is a biologically active substance for treating or diagnosing brain disease and is selected from the group consisting of small molecule drugs, peptides, antibodies, proteins, natural or modified ssDNA, dsDNA, RNA, siRNA, and ASO.

4. The blood-brain barrier-penetrating drug delivery system of claim 1, wherein the chemical linker is selected from the group consisting of -CONH-, -C(=O)-, -NH-, -O-, =N-, -SS-, and -N(CH₃)- and connected to SL, and is selected from the group consisting of -CONH-, -C(=O)-, -NH-, -O-, =N-, -SS- and -N(CH₃)- and connected to X, and
a linkage between SL and X has a structure selected from the group consisting of -(CH₂)a-(NHCO)b-(CH₂)c-, - (CH₂)a-(CONH)b-(CH₂)c-, -(CH₂)a-(CO)b-(CH₂)c-, -(CH₂)a-(NH)b-(CH₂)c-, and -(CH₂)a-(O)b-(CH₂)c-, wherein a is an integer ranging from 0 to 10, b is an integer of 0 or 1, and c is an integer ranging from 0 to 10.

5. The blood-brain barrier-penetrating drug delivery system of claim 1, wherein the chemical linker is a cleavable linker that promotes release of the drug to be delivered after the drug is delivered to the brain.

6. The blood-brain barrier-penetrating drug delivery system of claim 1, wherein the chemical linker is bound to a hydroxyl group (-OH) of a lactose moiety of the sialyllactose.

7. A pharmaceutical composition for treating or diagnosing brain disease comprising the blood-brain barrier-penetrating drug system of claim 1.
